# EUROPEAN PATENT SPECIFICATION

(11) **EP 2 388 035 B1**
(45) Date of publication and mention of the grant of the patent: **09.01.2013**
(21) Application number: 10163648.8
(22) Date of filing: 21.05.2010
(51) Int. Cl.: A61M 11/06, A61M 16/14, A61M 16/20

(54) **Press-type medical aerosol generating device and pressing mechanism for the same**
Medizinische Druckvorrichtung zur Aerosolerzeugung und Druckmechanismus dafür
Dispositif de génération d'aérosol médical de type presse et son mécanisme de presse

(43) Date of publication of application: 23.11.2011
(73) Proprietor: Atlantean Corp., Chubay City, Hsin chu 30265 (TW)
(72) Inventor: Chen, Chun-Hung, 231, Xindian City, Taipei County (TW); Huang, Mu-Hua, 110, Taipei City (TW)
(74) Representative: 2K Patentanwälte Blasberg Kewitz & Reichel

(56) References cited:
- GB-A- 2 334 217
- US-A- 5 461 695
- US-A- 6 085 741
- US-A1- 2003 089 367

## Description

### BACKGROUND OF THE INVENTION

### Field of the Invention

The present invention relates to a medicine-dispensing device, and in particular to a press-type medical aerosol generating device and a pressing mechanism for the same.

### Description of Prior Art

According to statistical data, over 70 percent of the human diseases are resulted from environmental conditions, and the environmental conditions have a great influence on the respiratory system of a human body. Thus, it is clinically proved that diseases of the respiratory tract can be cured by proper medicines, and the most common way is to dispense pill-like, capsule-like or liquid medicines through the mouth. However, before these medicines are assimilated by the human body, they will be decomposed by the alimentary canal. Thus, other ways of dispensing medicines through the mouth, nose, and lungs are developed, which is called aerosol therapy. To this end, a nebulizer is used to generate a medical aerosol for inhalation by a patient. The medical aerosol directly enters the bronchus and then spreads into the pulmonary alveolus, so that its therapeutic effect is more efficient and direct than that of oral medicine.

The conventional nebulizer includes a cylinder, an aerosol-generating assembly in communication with the bottom of the cylinder, a T-shaped tube in communication with the top of the cylinder, a mouth pipe and a buffer conduit in communication with both ends of the T-shaped tube respectively. Liquid medicament is received in the cylinder in advance, and then it is aerosolized by the aerosol-generating assembly. The free end of the buffer conduit is used as an open space, so that the patient bites the mouth pipe to inhale the medical aerosol.

When the patient inhales, the medical aerosol enters the patient's respiratory tract through the T-shaped tube and the mouth pipe, and the patient can inhale external air to complement the insufficient amount of inhalation.

When the patient stops inhaling, the nebulizer still generates the medical aerosol continuously and the excessive aerosol may escape to the outside. On the other hand, when the patient expires, the gas expired from the patient's lungs through the mouth pipe and the buffer conduit to the outside may also blow away the medical aerosol, which causes the waste of the medical aerosol and the environmental pollution. As a result, the liquid medicament received in the cylinder cannot be absorbed by the patient sufficiently. Thus, in order to achieve a desired therapeutic effect, the amount of liquid medicament has to be increased, which inevitably increases the medical expense.

U. S patent application publication No. 2003/0089367 discloses an atomizer for nasal cavities. This atomizer employs a button 24 to serve as a switch. In other words, the button 24 only can be operated in an on-off mode. A user can control the atomizer to spray or not to spray, but can not adjust the volume and pressure of atomized liquid when the button 24 is being pressed. As a result, the atomized liquid which is sprayed out may be overdosed and child users may be scared by the unvarying spraying pressure.

Therefore, in order to overcome the above problems, the present Inventor proposes a reasonable and novel structure based on his delicate researches and expert experiments.

### SUMMARY OF THE INVENTION

The present invention is to provide a pressing mechanism for a medical aerosol generating device, whereby a user can progressively control the device to generate the medical aerosol or stop the generation to thereby reduce the waste of medicament and increase the medicament-delivering efficiency with abetter therapeutic effect.

To this end, the device has a medicament storage container and a nebulizer provided in the medicament storage container. The nebulizer has a compressed gas channel. The pressing mechanism includes:
a base having a gas conduit in communication with the compressed gas channel for allowing the compressed gas to pass through, and a pressure relief hole in communication with the gas conduit, a periphery of the base on one side of the pressure relief hole being formed with a pivot; and
a pressing member comprising an operating arm pivotally connected to the pivot, a valve extending from the operating arm and positioned to correspond to the pressure relief hole, and an elastic arm connected to the operating arm and abutting against the medicament storage container;
wherein the valve closes the pressure relief hole when the operating arm is turned to rotate, so that the compressed gas can flow through the gas conduit into the channel; and
wherein the elastic arm pushes the valve to open the pressure relief hole when the operating arm is released, so that the compressed gas can flow through the gas conduit to escape from the pressure relief hole.

The present invention is to provide a press-type medical aerosol generating device, whereby a user can control the device to generate the medical aerosol or stop the generation to thereby reduce the waste of medicament and increase the medicament-delivering efficiency with a better therapeutic effect.

The present invention is to provide a press-type medical aerosol generating device, including:
a medicament storage container having a chamber for storing the medicament;
a nebulizer provided in the medicament storage container and having a compressed gas channel in communication with the chamber; and
a pressing mechanism, comprising:
   a base having a gas conduit in communication with the compressed gas channel for allowing the compressed gas to pass through, and a pressure relief hole in communication with the gas conduit, a periphery of the base on one side of the pressure relief hole being formed with a pivot; and
   a pressing member comprising an operating arm pivotally connected to the pivot, a valve extending from the operating arm and positioned to correspond to the pressure relief hole, and an elastic arm connected to the operating arm and abutting against the medicament storage container;
   wherein the valve closes the pressure relief hole when the operating arm is turned to rotate, so that the compressed gas can flow through the gas conduit into the channel, and the nebulizer aerosolizes the medicament received in the chamber; and
   wherein the elastic arm pushes the valve to open the pressure relief hole when the operating arm is released, so that the compressed gas can flow through the gas conduit to escape from the pressure relief hole to thereby stop the aerosolizing of the medicament

In comparison with prior art, the present invention has advantageous features as follows. The device can be controlled by a user to generate the medical aerosol or stop the generation. By operating the pressing mechanism, medical aerosols can be generated in the medicament storage container for inhalation by a patient. When the patient does not need to inhale the medical aerosol, the patient merely releases the pressing mechanism to cause the nebulizer to stop generating the medical aerosol. In this way, the waste of medicament can be reduced and the medicament-delivering efficiency can be increased with a better therapeutic effect.

### BRIEF DESCRIPTION OF THE DRAWINGS

FIG. 1 is an exploded perspective view of the present invention;
FIG 2 is an assembled perspective view of the present invention;
FIG. 3 is an assembled cross-sectional view of the present invention;
FIG 4 is a schematic view showing the generation of medical aerosols by the present invention;
FIG. 5 is a schematic view showing an operating state of the present invention for inhalation;
FIG. 6 is a schematic view showing another operating state of the present invention for exhalation; and
FIG. 7 is a schematic view showing that the present invention stops generating the medical aerosol.

### DETAILED DESCRIPTION OF THE INVENTION

The characteristics and technical contents of the present invention will be explained with reference to the accompanying drawings. However, the drawings are illustrative only but not used to limit the present invention.

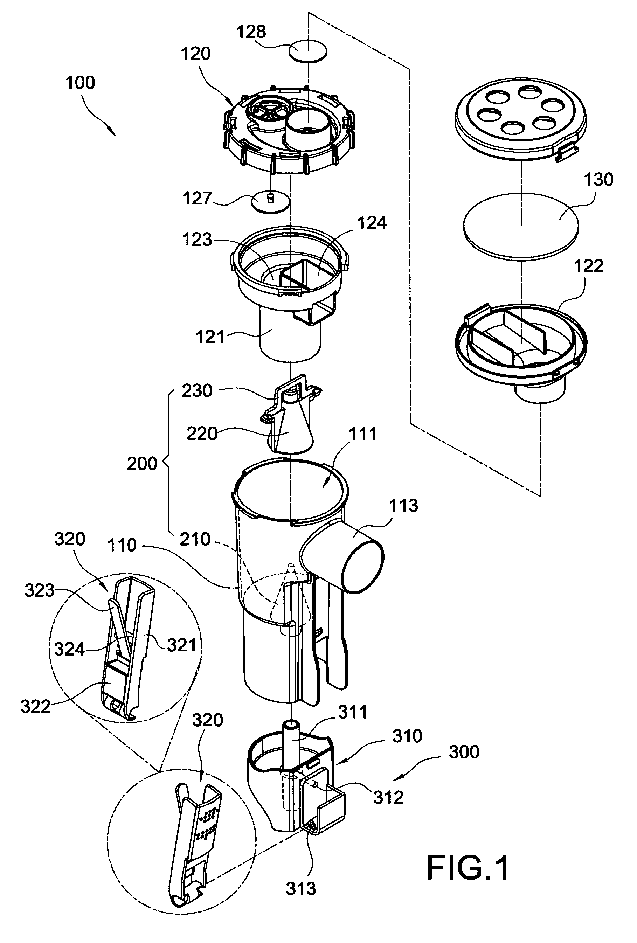

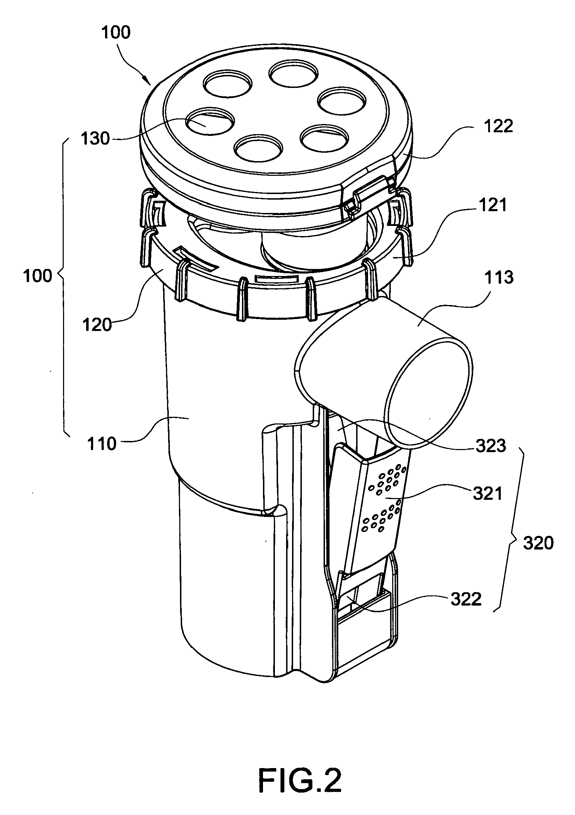

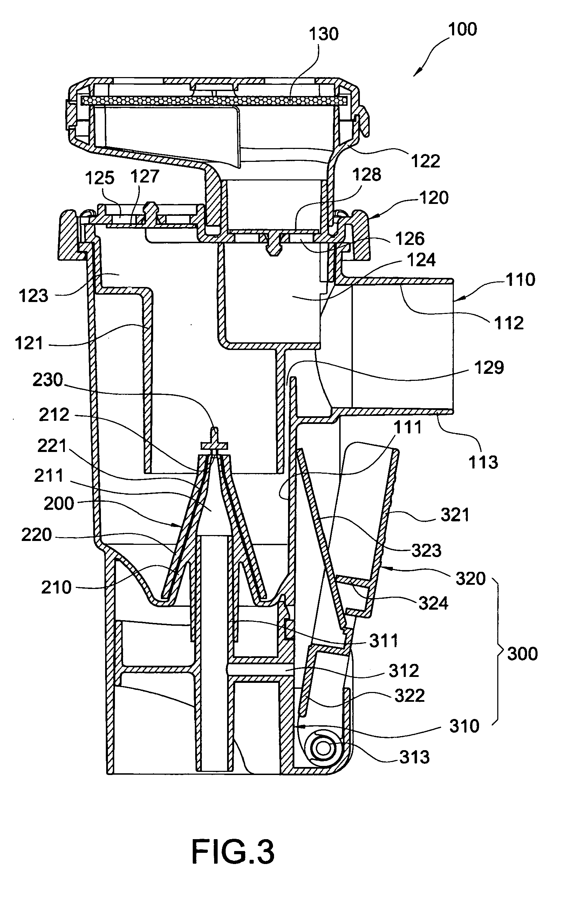

The present invention provides a press-type medical aerosol generating device and a pressing mechanism for the same. Please refer to FIGS. 1 to 3. The device includes a medicament storage container 100, a nebulizer 200 and a pressing mechanism 300.

The medicament storage container 100 includes a cylinder 110, a shroud 120, and a filtering piece 140.

The cylinder 110 is cylindrical but its shape is not limited thereto. The dimension and the shape are designed in such a manner that it can be gripped by a hand of a user. The interior of the cylinder 110 has a chamber 111 in communication with the top of the cylinder 110.

An upper portion of the outer peripheral surface of the cylinder 110 is provided with a mixture gas outlet 112 in communication with the chamber 111. The periphery of the mixture gas outlet 112 protrudes outwards to form a protruding pipe 113. The protruding pipe 113 is in communication with the mixture gas outlet 112.

The shroud 120 is inserted into the upper space of the chamber 111. The shroud 120 comprises a lower cover 121 and an upper cover 122 combined with the lower cover 121.

The interior of the lower cover 121 has an intake channel 123 and an exhaust channel 124 independent from each other. The intake channel 123 is arranged to correspond to the chamber 111 while the exhaust channel 124 is arranged to correspond to the mixture gas outlet 112. The top surface of the lower cover 121 is provided with an intake hole 125 in communication with the intake channel 123, and an exhaust hole 126 in communication with the exhaust channel 124.

Further, the lower cover 121 comprises a one-way intake valve 127 crossing the intake channel 123 for opening/closing the intake hole 125, and a one-way exhaust valve 128 crossing the exhaust channel 124 for opening/closing the exhaust hole 126. A mixture gas channel 129 is formed between the lower cover 121 and an inner wall of the cylinder 110 near the mixture gas outlet 112. The mixture gas channel 129 is in communication with the chamber 111 and the mixture gas outlet 112.

The upper cover 122 is arranged to correspond to the exhaust hole 126 and connected to the top surface of the lower cover 121. The filtering piece 130 is mounted in the upper cover 122 for filtering the gas through the exhaust hole 126. The upper cover 122 and the filtering piece 130 are optional components for filtering the gas exhaled by the patient.

The nebulizer 200 is inserted into the bottom of the cylinder 110. The nebulizer 200 is fixedly connected to the cylinder 110 by a press fit, but the way of achieving the connection there between is not limited thereto.

The nebulizer 200 comprises a conical post 210, a conical cover 220 and a stopper block 230.

The conical post 210 is formed by punching the bottom wall of the cylinder 110 toward the chamber 111. The recessed side of the conical post 210 is formed with a compressed gas channel 211. The top of the conical post 210 is provided with a compressed gas inlet 212 in communication with the chamber 111 and the compressed gas channel 211.

The conical cover 220 is hollow and configured to cover the outer protruding surface of the conical post 210. A fluid channel 221 is formed between the conical cover 220 and the conical post 210.

The stopper block 230 is positioned to correspond to the compressed gas inlet 212 and the fluid channel 221 above the conical cover 220 and the conical post 210.

The pressing mechanism 300 comprises a base 310 and a pressing element 320.

The base 310 comprises a gas conduit 311, a pressure relief hole 312 and a pivot 313.

The gas conduit 311 is inserted into the compressed gas channel 211. The pressure relief hole 312 is in communication with the gas conduit 311. The central line of the pressure relief hole 312 is perpendicular to the axial line of the gas conduit 311. The diameter of the pressure relief hole 312 is larger than that of the compressed gas inlet 212. The pivot 313 is formed on the outer periphery of the base 310 on the side of the pressure relief hole 312.

The pressing element 320 comprises an operating arm 321, a valve 322 and an elastic arm 323. The operating arm 321 is pivotally connected to the pivot 313. The valve 322 extends from the operating arm 321 and is positioned to correspond to the pressure relief hole 312 for opening/closing the pressure relief hole 312. The elastic arm 323 is connected to the operating arm 321 and abuts against the cylinder 110. The operating arm 321, the valve 322 and the elastic arm 323 can be integrally formed into one body to thereby shorten the manufacturing process and reduce the production cost with a better structural strength. Alternatively, the elastic arm 323 can be assembled with the operating arm 321. The operating arm 321 is formed with a pressing rod 324 for pressing the outer surface of the elastic arm 323.

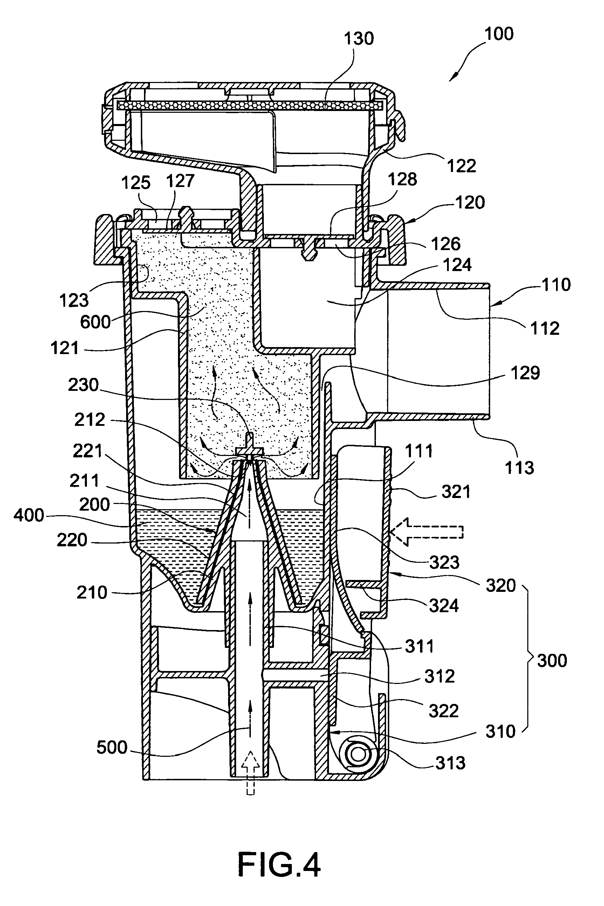

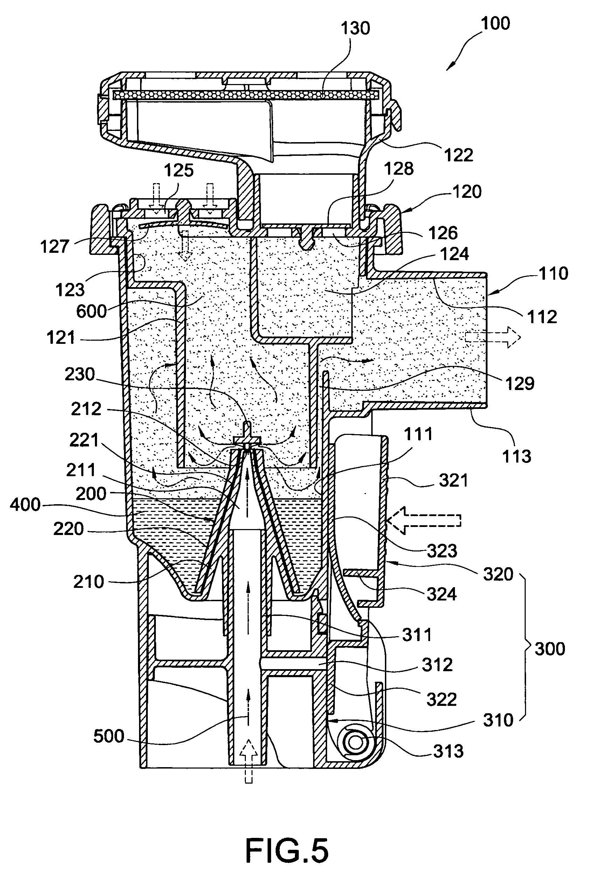

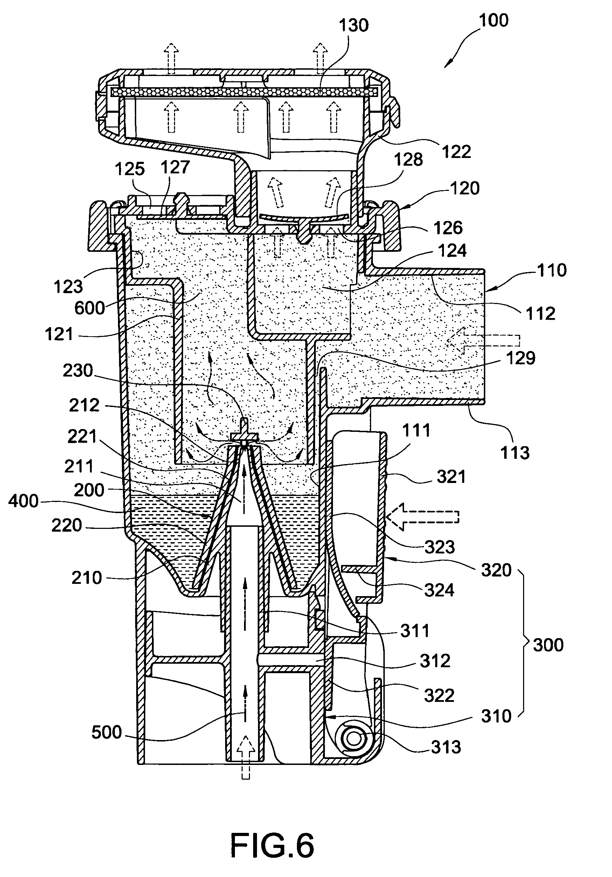

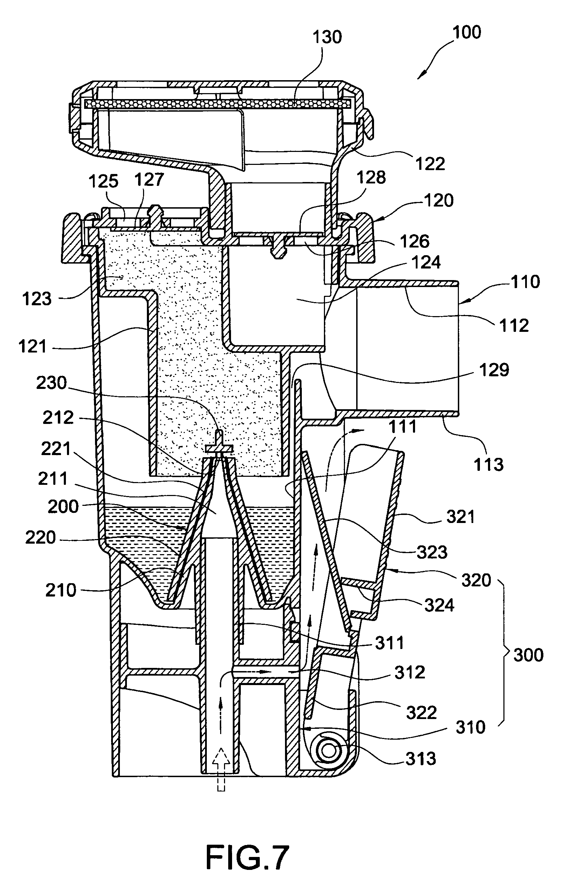

Please refer to FIGS. 4 to 7, which show the generation of a medical aerosol by the device of the present invention for inhalation. First, a medicament 400 is filled in the chamber 111 for curing diseases of a human body. The level of the medicament 400 is not higher than that of the compressed gas inlet 212 and the medicament 400 can flow into the fluid channel 221.

A compressed gas 500 is filled into the gas conduit 311. In general, compressed oxygen can be used as the compressed gas 500. The compressed gas 500 flows in the gas conduit 311 and the compressed gas channel 211. When the patient needs to inhale the medicament, the patient can turn the operating arm 321 to rotate with respect to the pivot 313, thereby causing the valve 322 to close the pressure relief hole 312. At this time, the pressing rod 324 presses the outer surface of the elastic arm 323 to generate an elastic deformation.

When the valve 322 closes the pressure relief hole 312, the compressed gas 500 can only flow through the gas conduit 311 into the compressed gas channel 211. Then, the compressed gas 500 flows into the compressed gas inlet 212 via the compressed gas channel 211 to generate a Venturi effect, whereby the compressed gas 500 forces the medicament 400 to flow out of the fluid channel 221.

The compressed gas 500 first subdivides the medicament 400, and the subdivided medicament 400 is ejected to the stopper block 230 under the action of the compressed gas 500. As a result, the subdivided medicament 400 is aerosolized and mixed with the compressed gas 500 to form a medical aerosol 600. Most of the medical aerosol 600 is filled in the intake channel 123.

The patient bites the protruding pipe 113 and inhales the medical aerosol 600 in the intake channel 123 through the chamber 111, the mixture gas channel 129 and the mixture gas outlet 112 into his lungs. The breathing-in of the patient causes the one-way intake valve 127 to open the intake bole 125 while causing the one-way exhaust valve 128 to tightly close the exhaust hole 126.

At this time, external air flows into the intake hole 125 to complement the insufficient amount of inhalation. Further, the external air guides most of the medical aerosol 600 to the mixture gas outlet 112 for halation by the patient. Thus, the patient may not inhale too much air, so that the efficiency in inhaling the medicament 400 can be increased and the period for therapy can be shortened.

After the patient inhales the medicament 400, the patient must exhale the waste gas from his lungs to the outside. The breathing-out of the patient causes the one-way exhaust valve 128 to open the exhaust hole 126. The waste gas exhaled by the patient can be exhausted to the outside through the mixture gas outlet 112 and the exhaust channel 124. The waste gas is filtered by the filtering piece 130, thereby preventing viruses or bacteria in the waste gas from diffusing to the outside.

At this time, since the breathing-in of the patient is stopped, the one-way intake valve 127 closes the exhaust hole 126 again, thereby preventing the medical aerosol 600 from escaping to the outside. Thus, the waste of medicament 400 can be avoided. Furthermore, since the cross-sectional area of the mixture gas channel 129 is much smaller than that of the exhaust channel 124, it is difficult for the waste gas to enter the chamber 111, and most of the waste gas can only be exhausted to the outside via the exhaust channel 124.

When the patient intends to stop inhaling the medical aerosol 600, the patient only needs to release the operating arm 321. The deformed elastic arm 323 restores its original shape to elastically pressing the pressing rod 324, thereby separating the valve 322 from the pressure relief hole 312 to open the pressure relief hole 312.

When the pressure relief hole 312 is opened, since the diameter of the pressure relief hole 312 is much larger than that of the compressed gas inlet 212, the compressed gas 500 can be dissipated to the outside through the pressure relief hole 312 without centering the compressed gas inlet 212. In this way, the generation of the medical aerosol 600 can be stopped.

The present invention is very convenient in use because the patient can inhale the medical aerosol 600 by operating the pressing mechanism 300 and stop the generation of the medical aerosol 600 by releasing the pressing mechanism 300. Thus, the waste of the medicament 400 and the medical aerosol 600 can be reduced. Also, the medicament-delivering efficiency can be increased with a better therapeutic effect. During the inhalation therapy, even the patient exhales, the medical aerosol 600 is not exhausted from the chamber 111, thereby saving the medicament 400 and the medical aerosol 600. Furthermore, the waste gas exhaled by the patient is filtered by the filtering piece 130, so that the viruses or bacteria in the waste gas can be prevented from spreading to the outside.

## Claims

1. A press-type medical aerosol generating device, including:
a medicament storage container (100) having a chamber (111) for storing medicament;
a nebulizer (200) provided in the medicament storage container (100) and having a compressed gas channel (211) in communication with the chamber (111); and
a pressing mechanism (300), comprising:
a base (310) having a gas conduit (311) in communication with the compressed gas channel (211) for allowing compressed gas to pass through, and a pressure relief hole (312) in communication with the gas conduit (311), a periphery of the base (310) on one side of the pressure relief hole (312) being formed with a pivot (313); and
a pressing member (320) comprising an operating arm (321) pivotally connected to the pivot (313), a valve (322) extending from the operating arm (321) and positioned to correspond to the pressure relief hole (312);
wherein the valve (322) closes the pressure relief hole (312) when the operating arm (321) is turned to rotate, so that the compressed gas can flow through the gas conduit (311) into the compressed gas channel (211), and the nebulizer (200) aerosolizes the medicament received in the chamber (111); and
wherein an elastic arm (323) pushes the valve (322) to open the pressure relief hole (312) when the operating arm (323) is released, so that the compressed gas can flow through the gas conduit (311) to escape from the pressure relief hole (312) to stop the aerosolizing of the medicament
**characterised in that** the elastic arm (323) is elastically connected to the operating arm (321) and abuts against the medicament storage container (100), the medicament storage container (100) comprises a cylinder (110) having the chamber (111), and a shroud (120) connected to one end of the cylinder (110), the nebulizer (200) comprises a conical post (210) formed on the cylinder (110), the compressed gas channel (211) is formed on a recessed side of the conical post (210), and the conical post (210) is formed with a compressed gas inlet (212) in communication with the chamber (111) and the compressed gas channel (211), the nebulizer (200) further comprises a stopper block (230) and a conical cover (220) provided at the periphery of the conical post (210), a fluid channel (221) is formed between the conical cover (220) and the conical post (210), and the stopper block (230) is arranged to correspond to the compressed gas inlet (212) and the fluid channel (221).

2. The press-type medical aerosol generating device according to claim 1, wherein the shroud (120) comprises a lower cover (121), the lower cover (121) having an intake channel (123) and an exhaust channel (124) independent from each other.

3. The press-type medical aerosol generating device according to claim 2, wherein the cylinder (110) is provided with a mixture gas outlet (112) in communication with the chamber (111), the intake channel (123) is arranged to correspond to the chamber (111), and the exhaust channel (124) is arranged to correspond to the mixture gas outlet (112).

4. The press-type medical aerosol generating device according to claim 3, wherein a mixture gas channel (129) is formed between the lower cover (121) and the cylinder (110) in communication with the chamber (111) and the mixture gas outlet (112).

5. The press-type medical aerosol generating device according to claim 2, wherein the lower cover (121) is provided with an intake hole (125) in communication with the intake channel (123) and an exhaust hole (126) in communication with the exhaust channel (124), and the lower cover (121) comprises a one-way intake valve (127) crossing the intake channel (123) for opening/closing the intake hole (125), and a one-way exhaust valve (128) crossing the exhaust channel (124) for opening/closing the exhaust hole (126).

6. The press-type medical aerosol generating device according to claim 5, wherein the medicament storage container (100) further includes a filter piece (130) positioned to correspond to the exhaust hole (126) for filtering the exhaust hole (126), the shroud (120) further comprises an upper cover (122) positioned to correspond to the exhaust hole (126) and connected to the lower cover (121), and the upper cover (122) is configured to accommodate the filtering piece (130).

7. The press-type medical aerosol generating device according to claim 1, wherein a diameter of the pressure relief hole (312) is larger than that of the pressured gas inlet (212).

8. The press-type medical aerosol generating device according to claim 1, wherein the operating arm (321) is formed with a pressing rod (324) for pressing an outer surface of the elastic arm (323).

9. The press-type medical aerosol generating device according to claim 1, wherein a central line of the pressure relief hole (312) is perpendicular to an axial line of the gas conduit (311).

## Patentansprüche

1. Ein medizinisches Aerosol erzeugendes Spray- Gerät - in Drücker-Konfiguration, enthaltend:
ein Medikamenten- Vorratsbehälter (100) mit einer Kammer (111) zum Speichern von Medikamenten;
einen Zerstäuber (200), der in dem Medikamenten- Vorratsbehälter (100) enthalten ist und einen Druckgas- Kanal (211) hat, der In Verbindung mit der Kammer (111) steht; und einem Drück- Mechanismus (300),
umfassend folgende Komponenten:
eine Basis (310) mit einer Gasleitung, (311) die in Verbindung mit dem Druckgas- Kanal (211) steht, um das Durchlaufen von Druckgas zu ermöglichen, und ein Druckentlastungsloch (312), das in Verbindung mit der Gasleitung (311) steht, eine Peripherie der Basis (310) auf einer Seite des Druckentlastungsloches (312), die mit einer Schwenkachse (313) ausgebildet ist, und
ein Drücker (320), beinhaltend einen Betätigungsarm (321), der schwenkbar ist und mit der Schwenkachse (313) verbunden ist, ein Ventil (322) das sich von dem Betätigungsarm (321) erstreckt und so positioniert ist, um mit dem Druckentlastungsloch (312) verbunden zu sein;
wobei das Ventil (322) das Druckentlastungsloch (312) schließt wenn der Betätigungsarm (321) gedreht wird, um zu rotieren, sodass das komprimierte Gas durch die Gasleitung (311) in den Druckgas- Kanal (211) strömen kann, und der Zerstäuber (200) das Medikament, welches in der Kammer (111) empfangen wird, zerstäubt, und
wobei ein elastischer Arm (323) das Ventil (322) schiebt, um das Entlastungsloch (312) zu öffnen, wenn der Betätigungsarm (323) entlastet wird, sodass das komprimierte Gas durch die Gasleitung (311) strömen kann, um aus dem Druckentlastungsloch (312) zu entweichen, um die Zerstäubung des Medikaments zu beenden
**dadurch gekennzeichnet, dass** der elastische Arm (323) elastisch mit dem Betätigungsarm (321) verbunden ist, und an dem Medikamenten-Vorratsbehälter (100) anliegt, der Medikamenten- Vorratsbehälter (100) einen Zylinder (110) mit der Kammer (111) umfasst und eine Ummantelung (120) verbunden mit einem Ende des Zylinders (110), der Zerstäuber (200) einen Konischen Stab (210) umfasst, der am Zylinder (110) ausgeprägt ist, der Druckgas- Kanal (211) an einer vertieften Seite des Konischen Stabes (210) angebracht ist und der konische Stab (210) ist mit einem Druckgas Einlass (212) gebaut ist welcher in Verbindung mit der Kammer (111) und dem Druckgas- Kanal (211) steht, der Zerstäuber (200) weiterhin einen Stopperblock (230) und eine kegelförmige Abdeckung (220) an der Peripherie des Konischen Stabes (210) umfasst, ein Flüssigkeitskanal (221) zwischen dem konischen Deckel (220) und dem konischen Stab (210) ausgeprägt ist und der Stopperblocker (230) derart angeordnet ist, dass er mit dem Druckgaseinlass (212) und dem Flüssigkeitskanal (221) entsprechend korrespondiert.

2. Ein medizinisches Aerosol erzeugendes Spray- Gerät in - Drücker-Konfiguration nach Anspruch 1, wobei die Ummantelung (120) eine untere Abdeckung (121)umfasst, die untere Abdeckung (121) einen Einlasskanal (123) und einen Auslasskanal (124) hat, welche unabhängig voneinander sind.

3. Ein medizinisches Aerosol erzeugendes Spray- Gerät in Drücker-Konfiguration nach Anspruch 2, wobei der Zylinder (110) mit einem Misch-Gasauslass (112) versehen ist, der in Verbindung mit der Kammer (111) steht, es ist vorgesehen, dass der Einlasskanal (123) und die Kammer (111) auf einander abgestimmt sind, und der Auslasskanal (124) mit dem Misch- Gasauslass (112) korrespondiert.

4. Ein medizinisches Aerosol erzeugendes Spray- Gerät in Drücker-Konfiguration nach Anspruch 3, wobei ein Misch- Gaskanal (129) zwischen der unteren Abdeckung (121) und dem Zylinder (110) ausgeprägt ist und in Verbindung steht mit der Kammer (111) und dem Mlsch- Gasauslass (112).

5. Ein medizinisches Aerosol erzeugendes Spray- Gerät in Drücker-Konfiguration nach Anspruch 2, wobei die untere Abdeckung (121) mit einem Einlassloch (125) versehen ist, welches in Verbindung mit dem Einlasskanal (123) ist, und ein Auslassloch (126) in Verbindung mit dem Auslasskanal (124) ist, und die untere Abdeckung (121) ein Einweg-Einlassventil (127) umfasst, das über dem Ansaugkanal (123) liegt, um das Einlassloch (125) zu öffnen oder zu schießen, und ein Einweg-Auslassventil (128), welches so am Abgaskanal (124), angebracht ist dass es das Abgasloch (126) öffnen oder schließen kann.

6. Ein medizinisches Aerosol erzeugendes Spray- Gerät in Drücker- - Konfiguration nach Anspruch 5, wobei der Medikamenten- Vorratsbehälter (100) ferner ein Filterstück (130) enthält, dass korrespondierend zum Abgasloch (126) angeordnet ist zur Filterung des Gases aus dem Abgasloch, wobei die Ummantelung (120) außerdem eine Obere Abdeckung (122) umfasst, die so positioniert ist, um zum Abgasloch (126) zu korrespondieren, und mit dem unteren Deckel (121) verbunden ist, und die obere Abdeckung (122) so konstruiert ist, dass das Filterstück (130) untergebracht wird.

7. Ein medizinisches Aerosol erzeugendes Spray- Gerät in Drücker- - Konfiguration nach Anspruch 1, wobei ein Durchmesser des Druckentlastungsloches (312) größer ist als der des Druckgaseinlasses (212).

8. Ein medizinisches Aerosol erzeugendes Spray- Gerät in Drücker-Konfiguration nach Anspruch 1, wobei der Betätigungsarm (321) mit einer Druckstange (324) versehen ist, um die Außenfläche des elastischen Arms (323) zu drücken.

9. Ein medizinisches Aerosol erzeugendes Spray- Gerät - in Drücker-Konfiguration nach Anspruch 1, wobei eine Mittellinie des Druckentlastungsloch (312) senkrecht zu einer axialen Linie der Gasleitung (311) steht.

## Revendications

1. Dispositif de génération d'aérosol médical de type à pression, comprenant :
un contenant de stockage de médicament (100) ayant une chambre (111) pour stocker un médicament ;
un nébuliseur (200) disposé dans le contenant de stockage de médicament (100) et ayant un canal de gaz comprimé (211) en communication avec la chambre (111) ; et
un mécanisme de pression (300), comprenant :
une base (310) ayant un conduit de gaz (311) en communication avec le canal de gaz comprimé (211) pour permettre à du gaz comprimé de passer à travers celui-ci, et un trou de décharge de pression (312) en communication avec le conduit de gaz (311), une périphérie de la base (310) sur un côté du trou de décharge de pression (312) comportant un pivot (313) ; et
un élément de pression (320) comprenant un bras d'actionnement (321) relié de manière pivotante au pivot (313), un clapet (322) s'étendant à partir du bras d'actionnement (321) et positionné pour correspondre au trou de décharge de pression (312) ;
le clapet (322) fermant le trou de décharge de pression (312) lorsque le bras d'actionnement (321) est amené à tourner, de telle sorte que le gaz comprimé peut s'écouler à travers le conduit de gaz (311) dans le canal de gaz comprimé (211), et le nébuliseur (200) nébulise le médicament reçu dans la chambre (111) ; et
un bras élastique (323) poussant le clapet (322) pour ouvrir le trou de décharge de pression (312) lorsque le bras d'actionnement (323) est libéré, de telle sorte que le gaz comprimé peut s'écouler à travers le conduit de gaz (311) pour s'échapper du trou de décharge de pression (312) afin d'arrêter la nébulisation du médicament,
**caractérisé par le fait que** le bras élastique (323) est relié élastiquement au bras d'actionnement (321) et vient en butée contre le contenant de stockage de médicament (100), le contenant de stockage de médicament (100) comprend un cylindre (110) ayant la chambre (111), et une coiffe (120) reliée à une extrémité du cylindre (110), le nébuliseur (200) comprend un plot conique (210) formé sur le cylindre (110), le canal de gaz comprimé (211) est formé sur un côté renfoncé du plot conique (210) et le plot conique (210) comporte une entrée de gaz comprimé (212) en communication avec la chambre (111) et le canal de gaz comprimé (211), le nébuliseur (200) comprend en outre un bloc bouchon (230) et un capuchon conique (220) disposé à la périphérie du plot conique (210), un canal de fluide (221) est formé entre le capuchon conique (220) et le plot conique (210), et le bloc bouchon (230) est agencé pour correspondre à l'entrée de gaz comprimé (212) et au canal de fluide (221).

2. Dispositif de génération d'aérosol médical de type à pression selon la revendication 1, dans lequel la coiffe (120) comprend un couvercle inférieur (121), le couvercle inférieur (121) ayant un canal d'admission (123) et un canal d'échappement (124) indépendants l'un de l'autre.

3. Dispositif de génération d'aérosol médical de type à pression selon la revendication 2, dans lequel le cylindre (110) comporte une sortie de gaz de mélange (112) en communication avec la chambre (111), le canal d'admission (123) est agencé pour correspondre à la chambre (111) et le canal d'échappement (124) est agencé pour correspondre à la sortie de gaz de mélange (112).

4. Dispositif de génération d'aérosol médical de type à pression selon la revendication 3, dans lequel un canal de gaz de mélange (129) est formé entre le couvercle inférieur (121) et le cylindre (110) en communication avec la chambre (111) et la sortie de gaz de mélange (112).

5. Dispositif de génération d'aérosol médical de type à pression selon la revendication 2, dans lequel le couvercle inférieur (121) comporte un trou d'admission (125) en communication avec le canal d'admission (123) et un trou d'échappement (126) en communication avec le canal d'échappement (124), et le couvercle inférieur (121) comprend un clapet d'admission antiretour (127) traversant le canal d'admission (123) pour ouvrir/fermer le trou d'admission (125), et un clapet d'échappement antiretour (128) traversant le canal d'échappement (124) pour ouvrir/fermer le trou d'échappement (126).

6. Dispositif de génération d'aérosol médical de type à pression selon la revendication 5, dans lequel le contenant de stockage de médicament (100) comprend en outre un élément filtre (130) positionné pour correspondre au trou d'échappement (126) pour la filtration du trou d'échappement (126), la coiffe (120) comprend en outre une couvercle supérieur (122) positionné pour correspondre au trou d'échappement (126) et relié au couvercle inférieur (121), et le couvercle supérieur (122) est configuré pour recevoir l'élément filtre (130).

7. Dispositif de génération d'aérosol médical de type à pression selon la revendication 1, dans lequel un diamètre du trou de décharge de pression (312) est plus grand que celui de l'entrée de gaz comprimé (212).

8. Dispositif de génération d'aérosol médical de type à pression selon la revendication 1, dans lequel le bras d'actionnement (321) comporte une tige de pression (324) pour presser sur une surface externe du bras élastique (323).

9. Dispositif de génération d'aérosol médical de type à pression selon la revendication 1, dans lequel un axe centrale du trou de décharge de pression (312) est perpendiculaire à une ligne axiale du conduit de gaz (311).
